# EUROPÄISCHE PATENTSCHRIFT

(11) **EP 3 345 630 B1**
(45) Veröffentlichungstag und Bekanntmachung des Hinweises auf die Patenterteilung: **19.02.2025**
(21) Anmeldenummer: 18153478.5
(22) Anmeldetag: 09.10.2014
(51) Int. Cl.: A61L 27/18, A61L 27/16, A61L 27/56, A61L 27/50, A61F 2/30, A61F 2/34, A61F 2/38, A61F 2/44, A61F 2/46, A61F 2/36

(54) **POLYMER FÜR EINEN GELENKSPACER, IMPLANTATE UND/ODER ALS SEHNEN- ODER BÄNDERERSATZ**
POLYMER FOR A JOINT SPACER, IMPLANTS AND/OR AS ARTIFICIAL TENDON OR LIGAMENT
POLYMÈRE POUR UN ÉCARTEUR ARTICULAIRE, DES IMPLANTS ET/OU EN TANT QU'ÉLÉMENT DE REMPLACEMENT DE LIGAMENT OU DE TENDON

(30) Priorität: 11.10.2013 DE 102013016899; 19.11.2013 DE 202013010444 U
(43) Veröffentlichungstag der Anmeldung: 11.07.2018
(62) Teilanmeldung aus: 14809758.7
(73) Patentinhaber: Revomotion GmbH, 50937 Köln (DE)
(72) Erfinder: Jansen, Josef, 51429 Bergisch Gladbach (DE)

(56) Entgegenhaltungen:
- WO-A2-98/20939
- DE-A1- 10 243 966
- US-A- 6 140 452
- US-A1- 2007 027 285
- US-A1- 2013 218 275
- HERRLINGER ET AL.: "Houben-Weyl, Methoden der organischen Chemie", vol. 24/2, 7 January 1963, GEORG THIEME VERLAG, Stuttgart, article E.MÜLLER: "Kautschukelastische Stoffe", pages: 79 - 81, XP055481377
- VIEWEG ET AL.: "Kunststoff Handbuch", vol. VII, 1966, CARL HANSER VERLAG, München, article ELLEGAST, KONRAD: "Wasservernetzte Produkte", pages: 270 - 273, XP055481374
- ONG ET AL: "New Biomaterials for Orthopedic implants", ORTHOPEDIC RESEARCH AND REVIEWS, vol. 2015-7, 8 September 2015 (2015-09-08), pages 107 - 130, XP055481290, DOI: https://doi.org/10.2147/ORR.S63437
- JONES E ET AL: "Compliant-layer tibial bearing inserts: Friction testing of different materials and designs for a new generation of prostheses that mimic the natural joint", PROCEEDINGS OF THE INSTITUTION OF MECHANICAL ENGINEERS.JOURNAL OF ENGINEERING IN MEDICINE. PART H, MECHANICAL ENGINEERING PUBLICATIONS LTD, LONDON, GB, vol. 222, no. 8, January 2008 (2008-01-01), pages 1197 - 1208, XP009505574, ISSN: 0954-4119, DOI: 10.1243/09544119JEIM442
- "PUR Elastomere", XP055481270, Retrieved from the Internet <URL:http://www.mattec.at/Polyurethaninfo.htm> [retrieved on 20180605]

## Beschreibung

Die vorliegende Erfindung betrifft ein biokompatibles Polymer für Gelenkspacer, insbesondere einen Kniespacer und einen Hüftspacer, Implantate und/oder als Sehnen- oder Bänderersatz bestehend aus einem vernetzten Polyurethan, wobei das Polyurethan Polyurethanharnstoff oder Polyharnstoff ist.

Von Osteoarthrose, dem vorzeitigen Verschleiß knorpeliger Gelenkflächen, sind weltweit viele Millionen Menschen betroffen, der größte Teil davon an den Knien und an der Hüfte. Als Alternative zu den bisherigen mit vielfältigen Nachteilen verbundenen künstlichen Gelenken (Endoprothesen) bieten sich für das Knie elastische, scheibenförmige Abstandshalter, sogenannte "Kniespacer" an. Diese lassen sich mittels eines kleinen Hautschnitts nach der Entfernung des (Rest-) Meniskus, der Glättung der Gelenkflächen und einer sorgfältigen Größenauswahl in den Knieinnenraum einführen. Sie ersetzen im Gelenk den abgeriebenen Knorpel und den beschädigten Meniskus. Die Kniespacer stellen den natürlichen Gelenkspalt wieder her und können so auch Abwinklungen, wie beispielsweise X- oder O-Fehlstellungen (Valgus, Varus) des Beines, korrigieren.

Die natürlichen medialen (innen) und lateralen (außen) Menisken sind halbmondförmige Faserknorpelgewebe, die im Querschnitt keilförmig sind und über das Vorder- und Hinterhorn im Tibiaplateau verankert sind. Sie vergrößern die Kontaktfläche zwischen dem Tibiaplateau und den Gelenkrollen (Kondylen) des Femurs (Oberschenkelknochen).

Gelenkspacer bezeichnen allgemein Abstandshalter, die auch in anderen Gelenken, wie beispielsweise der Hüfte, der Schulter, dem Fuß, der Hand oder der Wirbel des menschlichen Körpers eingesetzt werden können. Gelenkspacer beinhalten hier auch sogenannte "Plugs" oder flache Stöpsel, welche nur einen kleinen Teil der Knorpelfläche eines Gelenks ersetzen können. Zudem sind Abstandshalter als Gleit- und Dämpfungselemente für Endoprothesen einsetzbar. Bei Kniespacern wird zwischen verschiedenen Ausführungsformen unterschieden. Der Meniskusspacer ersetzt den Meniskus und abgelaufenen Knorpel der Gelenkflächen und das Meniskusimplantat lediglich den Meniskus. Der Gelenkflächenersatz oder Gelenkflächenspacer gleicht den teilweise abgelaufenen Knorpel bzw. den beginnenden Verschleiß des Knorpels der Gelenkflächen ohne Ersatz des Meniskus aus.

Es ist bekannt, dass Kniespacer aus unterschiedlichen Materialien und Polymeren bestehen können. Dabei sind Polyurethane aufgrund ihrer hohen mechanischen Festigkeit und hohen Abriebfestigkeit sehr gut geeignet. Grundsätzlich kann bei Polyurethanen zwischen aromatischen und aliphatischen Polyurethanen unterschieden werden. Aromatische Polyurethane besitzen üblicherweise die größere mechanische Festigkeit und scheinen daher besser geeignet für die hohen Belastungen, die in einem Kniespacer während der Beugung und der Streckung auftreten. Aromatische Polyurethane haben gegenüber aliphatischen jedoch den Nachteil, dass sie "vergilben" können, also einen gewissen Alterungseffekt zeigen. Zudem stehen aromatische Polyurethane in der Diskussion, dass ihre potenziellen Abbauprodukte ein kanzerogenes Risiko besitzen können.

Kniespacer haben sich bis heute noch nicht durchsetzen können, weil die Implantate häufig brechen, aus dem Gelenkspalt des Knies herausrutschen oder Bewegungseinschränkungen auftreten. Insbesondere sind derzeit keine Kniespacer mit physiologischer Dämpfung bei gleichzeitig ausreichender mechanischer Festigkeit und Dauerfestigkeit bekannt. Insbesondere eine physiologische Dämpfung ist jedoch erstrebenswert, da sie zu einer Schmerzfreiheit des Patienten führt.

Aus US 6 140 452 A, US 2013/218275 A1, US 2007/027285 A1, E.Müller: "Kautschukelastische Stoffe" (In: Herrlinger et al.: "Houben-Weyl, Methoden der organischen Chemie", 7. Januar 1963 (1963-01-07), Georg Thieme Verlag, Stuttgart, XP055481377, Bd. 24/2, Seiten 79-81), Ellegast, Konrad: "Wasservernetzte Produkte" (In: Vieweg et al.: "Kunststoff Handbuch", 1966, Carl Hanser Verlag, München, XP055481374, BD. VII, Seiten 270-273), ONG ET AL: "New Biomaterials for Orthopedic implants" ("ORTHOPEDIC RESEARCH AND REVIEWS, Bd. 2015-7, 8. September 2015 (2015-09-08), Seiten 107-130; XP055481290; DOI: https.//doi.org/10.2147/ORR.S63437), JONES E ET AL: "Compliant-layer tibial bearing inserts: Friction testing of different materials and designs for a new generation of prostheses that mimic the natural joint", (PROCEEDINGS OF THE INSTITUTION OF MECHANICAL ENGINEERS.JOURNAL OF ENGINEERING IN MEDICINE. PART H, MECHANICAL ENGINEERING PUBLICATIONS LTD, LONDON, GB, Bd. 222, Nr. 8, Januar 2008 (2008-01), Seiten 1197-1208, XP009505574, ISSN: 09954-4119; DOI: 10.1243/09544119JEIM442),"PUR Elastomere", XP055481270, Gefunden im Internet:
URL:http://www.mattec.atZPolyurethaninfo.htm, DE 102 43 966 A1 und WO 98/20939 A2 sind weitere Materialien bekannt, die insbesondere für Gelenkspacer verwendet werden.

Es ist die Aufgabe der vorliegenden Erfindung, ein Polymer für einen haltbaren, ausreichend gedämpften und abriebfesten Gelenkspacer zu schaffen, der auch punktuell sehr hohe Lasten aufnehmen kann. Die aus dem Polymer gemachten Gelenkspacer sollten zudem eine möglichst hohe Kongruenz zwischen den artikulierenden Gelenkflächen erzielen und nicht aus dem Gelenkspalt herausrutschen können. Das Polymer soll auch für Implantate und/oder als Sehnen- oder Bänderersatz geeignet sein.

Diese Aufgabe wird durch das biokompatible Polymer nach Anspruch 1 gelöst. Erfindungsgemäß ist vorgesehen, dass zur Vernetzung des Polyurethans ein Vernetzungsreagenz eingesetzt wird, das zumindest anteilig Wasser aufweist.

Ein solches Polymer, insbesondere für einen Gelenkspacer zeigt vorteilhafterweise ein ausgeprägtes progressives Druck-Stauchungsverhalten. Mit zunehmender Stauchung nimmt nämlich die Elastizität überproportional ab und das Polymer wird mithin härter. Mit anderen Worten das Material ist hinsichtlich seiner Shore-Härte relativ weich und besitzt quer zur Stauchung gleichzeitig einen relativ hohen Elastizitätsmodul.

Außerdem eignen sich die beschriebenen Polymere für weitere Implantate des menschlichen Körpers, wie beispielsweise für Gefäße, Herzklappen und sonstige Ventile im menschlichen Körper sowie für Pumpenmembranen und -kammer in künstlichen Herzen und Herzunterstützungssystemen. Des Weiteren sind sie für ophthalmologische Implantate wie Intraokularlinsen oder künstliche Hornhäute geeignet.

In den Unteransprüchen sowie nachfolgend werden bevorzugte Ausgestaltungen der Erfindung beschrieben.

Nach einer ersten vorteilhaften Ausgestaltung ist vorgesehen, dass das Vernetzungsreagenz einen oder mehrere Kettenverlängerer aufweist. Ferner besitzt das Vernetzungsreagenz vorzugsweise ein oder mehrere Vernetzungsreagenzien.

Im Zusammenhang mit der Auswahl geeigneter Materialien bzw. Polymere für Gelenkspacer wird häufig die Shore-Härte herangezogen. Die in den Gelenken, besonders in Knie und Hüfte, auftretenden sehr hohen Belastungen können unter den bekannten für die Medizintechnik geeigneten Materialien nur durch Materialen mit Shore-A-Härten oberhalb von 85 aufgenommen werden. Für eine hohe Kongruenz und Dämpfung und letztlich damit Schmerzfreiheit für den Patienten wären Gelenkspacer aus solchen Materialien jedoch im Knie zu hart und daher weniger geeignet. Je weicher der Gelenkspacer, desto größer ist die Kontaktfläche zwischen Gelenkspacer und Gelenkfläche und desto kleiner die Spannung und damit auch der Abrieb im Gelenkspacer. Der Gelenkspacer sollte idealerweise in seiner Komplianz dem natürlichen Gelenkknorpel angenähert sein. Dies schließt den geschwächten arthrotischen Gelenkknorpel ein, so dass dieser nach Möglichkeit nicht weiter durch das Implantat geschädigt werden kann. Es wird ein Shore-A-Härte Bereich des Materials von 20 bis 77, vorzugsweise von 45 bis 72 angestrebt. Für sehr schwergewichtige Menschen, abhängig von der Größe der Tibiafläche bzw. des Gelenkspacers ab ca. 100-120 kg, kann darüber hinaus die Shore-A-Härte des Materials bis 85 liegen. Hierzu kommen als Materialien in erster Linie Elastomere oder thermoplastische Elastomere in Frage. Die bekannten flexiblen Polymere bzw. sonstigen Materialien in diesem Härtebereich können bei höheren Verformungen nicht genügend Spannung aufbauen, um den auftretenden Kräften in den Gelenken standzuhalten, d.h. die Tragfähigkeit des Lagermaterials reicht nicht aus. Es hat sich in Versuchsreihen jedoch gezeigt, dass Materialien, die ein ausgeprägt progressives Druck-Stauchungsverhalten aufzeigen, für Gelenkspacer geeignet sind. Materialien können nämlich bei gleicher Shore-Härte sehr unterschiedlich hohe Elastizitätsmodule aufweisen, und insbesondere bei einer vorgegebenen Dehnung oder Stauchung unterschiedlich hohe Spannungen aufbauen. Anders ausgedrückt wird unter einer vorgegebenen Last das Material bei gleicher Shore-A-Härte weniger oder mehr gestaucht.

Materialien, insbesondere Elastomere und thermoplastische Elastomere können bei Temperaturerhöhung, nach Aufnahme von Wasser und längerer, mehrmonatiger Lagerung in Wasser oder bei mehrfacher Belastung (belastungsinduzierte Entfestigung oder sogenannter Mullins-Effekt) erweichen (Geary C et al. Characterisation of Bionate polycarbonate polyurethanes for orthopaedic applications. J Mater Sci: Mater Med 19 (2008) 3355-3363). Zudem können sich durch Sterilisation die mechanischen Eigenschaften verändern. Weiterhin können sich Materialien bei sehr hohen Prüfgeschwindigkeiten verfestigen. Die in dieser Erfindung angegebenen Parameter beziehen sich daher auf Prüfbedingungen der Proben in einem konditionierten Zustand bei 37°C, nach Aufnahme von Wasser bzw. Gelenkflüssigkeit und mit den üblichen Prüfgeschwindigkeiten der einschlägigen Normen und in einem sterilisierten Zustand. Die Spannungswerte werden vorzugsweise nach dem 5. Zyklus ermittelt, um den Mullins Effekt auszugleichen, da sich die Größe der Hysterese insbesondere in den ersten Zyklen stark ändern kann. Zudem sollten die zuvor angegebenen Werte auch nach mindestens 5 monatiger Lagerung in Wasser erreicht bzw. überschritten werden. Die Materialien sollten also hydrolytisch möglichst stabil sein. Allgemeiner ausgedrückt, sollen die eingesetzten Materialien die Parameter auch nach längerer Implantation im Körper beibehalten.

Vorzugsweise besteht daher das Polymer zumindest teilweise aus einem Material, insbesondere einem Elastomer oder thermoplastischen Elastomer mit einer Shore-A Härte zwischen 20 und 77 und Zugspannungswerten bei Dehnung zwischen 20% und 60% , vorzugsweise bei 50% (im folgenden auch 50%-Zugspannung genannt), größer 3,8 N/mm², vorzugsweise größer 4,6 N/mm² und besonders bevorzugt größer 6 N/mm², und/oder einer Shore-A Härte bis 85 und Zugspannungswerten bei Dehnungen zwischen 20% und 60% , vorzugsweise bei 50%, größer 6 N/mm², vorzugsweise größer 7 N/mm² und besonders bevorzugt größer 8 N/mm². Angemerkt ist hier, dass unter dem Spannungswert bei einer bestimmten Dehnung nicht der Tangenten- oder Sekantenmodul verstanden wird. Das Material sollte außerdem bevorzugt kein Fließverhalten bis 50% Dehnung, vorzugsweise bis 70% Dehnung aufweisen.

Vorzugsweise weist das Material bei einer Shore-A Härte zwischen 20 und 77 Zugspannungswerte bei 100%-Dehnung (100%-Zugspannung) von mindestens 5 N/mm², vorzugsweise mindestens 6 N/mm² und besonders bevorzugt mindestens 7,5 N/mm², oder bei einer Shore-A Härte bis 85 Zugspannungswerte bei 100%-Dehnung von mindestens 7,5 N/mm², vorzugsweise mindestens 8,5 N/mm² und besonders bevorzugt mindestens 10,5 N/mm² auf.

Eine weitere Kenngröße für geeignete Materialien sind die Druckspannungen, auch wenn die Schädigung des Materials deutlich mehr von den auftretenden Zugspannungen hervorgerufen wird. Vorzugsweise besteht daher das Polymer ferner zumindest teilweise aus einem Material mit einer Shore-A Härte zwischen 20 und 77 und Druckspannungswerten bei Stauchungen zwischen 20% und 60%, vorzugsweise bei 50% (im folgenden auch 50%-Druckspannung genannt), größer 7,8 N/mm², vorzugsweise größer 9 N/mm² und besonders bevorzugt größer 10,5 N/mm², und/oder einer Shore-A Härte bis 85 und Druckspannungswerten bei Stauchungen zwischen 20% und 60%, vorzugsweise bei 50%, größer 10,5 N/mm², vorzugsweise größer 12 N/mm² und besonders bevorzugt größer 14 N/mm² bestehen.

Die zuvor genannten Spannungswerte können auch bereits bei niedrigeren Dehnungs- bzw. Stauchungswerten als den genannten Werten erreicht werden, auch wenn das Polymer oder der Gelenkspacer dann bei gleichen Belastungen nicht so stark zusammengestaucht wird.

Wie zuvor angedeutet, können weichere Materialien zumeist den in den Gelenken auftretenden Belastungen nicht standhalten. Neben den zuvor beschriebenen Zugspannungswerten bei 50%- oder auch 100%-Dehnung und den 50%-Druckspannungen ist die Weiterreißfestigkeit ein weiterer wichtiger Materialparameter. Je weicher das Material ist, desto höher sollte idealerweise die Weiterreißfestigkeit sein, mit anderen Worten soll sie umgekehrt proportional zur Shore-A-Härte stehen. Bezugswerte dieser Beziehung sind: bei einer Shore-A-Härte von 55 soll die Weiterreißfestigkeit größer 60N/mm (vorzugsweise größer 70 N/mm) sein, bei einer Shore-A-Härte von 75 größer 35 N/mm (vorzugsweise größer 40 N/mm). Über diese Bezugswerte soll ein linearer Verlauf über die Shore-A-Härte Bereiche bestehen. Die Weiterreißfestigkeiten beziehen sich auf Probenformen gemäß dem "Trouser Tear Test".

Als Materialien für die Gelenkspacer eignen sich biokompatible und elastische, dauerfeste Polymere und hier, wie bereits ausgeführt, insbesondere die Klasse der Polyurethane. Ferner sollen die Polymere möglichst frei sein von niedermolekularen Bestandteilen, wodurch die gewünschten 50%-Spannungswerte gesteigert werden und damit zu den bevorzugten Eigenschaften führen.

Polyurethane sind charakterisiert durch das Zusammenspiel von Hart- (gebildet aus einem Isocyanat mit niedermolekularen Kettenverlängerern) und (höhermolekularen) Weichsegmenten. Abhängig von ihrer Zusammensetzung zeigen Polyurethane ein sehr unterschiedliches Werkstoffverhalten, insbesondere im Spannungs-/Dehnungsverlauf.

Isocyanate werden in der Literatur vielfältig beschrieben und können grundsätzlich alle eingesetzt werden. Bevorzugt werden jedoch kompakte, wenig verzweigte und hochschmelzende Isocyanate wie Cyclohexandiisocyanat (CHDI), Naphthalin-1,5-Diisocyanat (NDI), oder Para-Phenylendiisocyanat (PPDI). Ferner eignen sich lineare, symmetrische Isocyanate wie Hexamethylendiisocyanat (HDI). Derartige Ausgangskomponenten begünstigen weiter das progressive Druck-Stauchungsverhalten, woraus sich die genannten Vorteile ergeben. Zudem sind diese Isocyanate besonders gut geeignet für dynamische Anwendungen. Die Gewichtsanteile dieser Isocyanate betragen bis zu 50% vorzugsweise 3% - 30% der Polymere. Diese bevorzugten Isocyanate können ferner mit weiteren aromatischen oder aliphatischen Isocyanaten gemischt werden. Den zuvor genannten Diisocyanaten oder Mischungen aus Diisocyanaten können auch Polyisocyanate hinzugefügt werden.

Nach einer bevorzugten Ausgestaltung der vorliegenden Erfindung ist vorgesehen, dass das Polyurethangemisch zumindest teilweise trans-1,4-Cyclohexandiisocyanat (CHDI), cis-CHDI oder Mischungen hiervon besitzt. Trans-CHDI ist dabei besonders bevorzugt, weil das Polymer dadurch eine besonders hohe Kristallinität aufweist und damit die bevorzugten Eigenschaften erhält. CHDI führt zudem zu den favorisierten aliphatischen Polyurethanen. Ferner ist vorgesehen, dass das Polyurethangemisch zumindest teilweise aus weiteren aliphatischen Isocyanaten besteht, insbesondere aus Dicyclohexylmethandiisocyanat (H₁₂MDI) oder einem Isomerengemisch hiervon, oder auch einem Gemisch aus CHDI und H₁₂MDI.

Im Folgenden werden eine Reihe von Formulierungen bzw. Komponenten angegeben, die alle für sich vorteilhafterweise zu den bevorzugten Eigenschaften führen.

Zur Erzielung des gewünschten progressiven Druck-Stauchungsverhaltens von Polyurethanen mit hohen Spannungswerten bei 50%-Dehnung oder Stauchung und niedriger Shore-A-Härte sind die Weichsegmente von entscheidender Bedeutung. Vorzugsweise kommen für das Polymer hydrolysefeste und biostabile Weichsegmente auf Basis von Polyolefinen wie insbesondere Polyisobutylen (PIB) oder Polybutadien (PB) in Frage. Ferner gelten auch Weichsegmente auf Basis von Polycarbonat (PC) und Polydimethylsiloxan (PDMS) als relativ hydrolysefest. Typische aus der Literatur bekannte bzw. kommerzielle, auch medizinische Polyester- oder Polyetherurethane sind für Langzeitimplantate nicht genügend biostabil und kommen daher für das Polymer weniger bzw. nicht bevorzugt in Frage, auch wenn sie in relativ kleinen Anteilen beigemischt werden können. Auch Silikon-Ether-Polyurethan Copolymere scheinen eine ungenügende Biostabilität zu haben.

Nach einer bevorzugten Ausgestaltung der vorliegenden Erfindung ist das Weichsegment des Polymers ein Polyisobutylen (PIB) bzw. ein bifunktionelles Polyisobutylen, bevorzugt ein hydroxylterminiertes (HO-PIB-OH) oder ein aminterminiertes (H₂N-PIB-NH₂) Polyisobutylen.

In einer anderen bevorzugten Ausgestaltung ist das Weichsegment Polybutadien (PB), bevorzugt OH-terminiertes und besonders bevorzugt OH-terminiertes hydrogeniertes Polybutadien. Es können jedoch auch aminterminierte Polybutadiene eingesetzt werden.

Die vorteilhaften Eigenschaften des Polymers werden besonders hoch, wenn das Weichsegment ausschließlich aus einem PIB oder PB oder auch aus einer Mischung dieser beiden Weichsegmente besteht.

Die Polyurethantypen des biokompatiblen Polymers sind vernetzt. Hierfür eignet sich vorzugsweise Wasser für die Erzielung der gewünschten Eigenschaften. Es können jedoch weitere Vernetzungsreagenzien wie z.B. 3-wertiges Glycol oder sonstige mehrwertige aus der Polyurethanchemie an sich bekannte Vernetzungsreagenzien eingesetzt werden.

Bevorzugte Isocyanate bei der Wasservernetzung sind HDI, NDI, PPDI oder CHDI. Bevorzugte Weichsegmente sind hierbei neben den zuvor genannten bevorzugten Weichsegmenten PIB und PB auch Polycarbonatdiol (PCD) und zudem gegebenenfalls als Kettenverlängerer Butandiol (BD) oder ein oder mehrere andere Kettenverlängerer. Vorzugsweise wird jedoch auf einen weiteren Kettenverlängerer verzichtet, also nur Wasser zur Vernetzung eingesetzt.

Bei Polyurethanen auf Basis von Polycarbonatdiolen wird in der Medizintechnik üblicherweise Polyhexamethylencarbonatdiol (C₆-PCD) eingesetzt. Diese sind ausschließlich aus jeweils sechs Methylengruppen (CH₂) zusammengesetzt. Um jedoch weichere Polyurethane zu erzielen, zugleich aber die vorteilhaften Materialeigenschaften zu erzielen, die für das Polymer besonderes gut geeignet sind, werden Polycarbonatdiol-Copolymertypen (Polyalkylen-Carbonatdiole) statt eines homogenen C₆-PCD eingesetzt, die aus den Einheiten Hexan (C₆), Pentan (Cs), Butan (C₄) oder Propan (Cs) aufgebaut sind. Vorzugsweise bestehen die Copolymertypen aus den Kombinationen mit C₆/C₅ oder C₆/C₄ oder C₄/C₃ Einheiten, in der chemischen Summenformel ausgedrückt mit 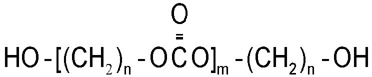 also mit n=6 oder 5; oder n=6 oder 4; oder n=4 oder 3. Zudem zeigen die Copolymertypen mit abnehmender Anzahl an Methylengruppen ein deutlich besseres Abriebverhalten.

Eine bevorzugte Formulierung sieht daher vor, dass das Polymer des Gelenkspacers zumindest teilweise Polycarbonatdiol-Copolymere enthält.

Besonders bevorzugte Komponenten des Polyurethansystem sind, wie bereits ausgeführt, die Isocyanate HDI, CHDI, PPDI oder NDI sowie als Weichsegmente Polyolefine, bevorzugt PIB oder PB und PCD, wobei bei PCD vorzugsweise die zuvor genannten Polycarbonatdiol-Copolymertypen eingesetzt werden. Hierbei wiederum können die Polyolefintypen PIB oder PB gemischt oder jeweils insbesondere mit dem PCD gemischt werden, um besonders abriebfeste Polyurethantypen zu erzielen. Der Anteil von PCD am Gesamtweichsegmentanteil beträgt bis zu 80%, vorzugsweise 5 bis 40%. Die Weichsegmente können ferner mit weiteren bekannten Weichsegmenten wie insbesondere Polydimethylsiloxan (PDMS) oder Polytetramethylenoxid (PTMO) gemischt werden. Auch eignet sich zur Erzielung der vorteilhaften Eigenschaften die Beimischung von Polydimethylsiloxan-Polycaprolacton-Blockcopolymeren. Die Anteile dieser Weichsegmente am Gesamtweichsegmentanteil betragen bis zu 50%, vorzugsweise 3 bis 35%.

Eine weitere vorteilhafte Formulierung sieht vor, dass das Polymer des Gelenkspacers als Weichsegmente ausschließlich hydroxyl- und/oder aminterminiertes Polyisobutylen, und/oder hydroxyl- und/oder aminterminiertes und bevorzugt hydroxylterminiertes hydrogeniertes Polybutadien, und/oder Polycarbonatdiol enthält.

Bei den zuvor genannten amin- oder hydroxylterminierten PIB kann es sich zudem um lineare oder verzweigte PIB's handeln. Bei den Polybutadienen können auch Copolymerisate wie Acrylnitril-Butadien-Styrol eingesetzt werden.

Ferner können auch die zuvor genannten Isocyanate mit anderen aromatischen oder aliphatischen bzw. cycloaliphatischen Isocyanaten gemischt werden, wie beispielsweise 3,3'-Dimethyl-4,4'-biphenylendiisocyanat (TODI).

Außerdem können in den zuvor aufgezeigten Formulierungen die genannten Kettenverlängerer auch in Kombination zweier oder mehrerer Kettenverlängerer und/oder Vernetzungsreagenzien (z. B. Glycol) in die Polymersynthese eingebracht werden.

Mit den zuvor aufgezeigten Ausgestaltungen des Materials, d.h. je nach genauer Zusammensetzung der einzelnen Komponenten können 50%-Zugspannungen bis 20 N/mm² in den genannten Shore-A-Härtebereichen erzielt werden.

Zur Verbesserung der Gleiteigenschaften bzw. zur Reduzierung des Reibwertes ist vorgesehen, dass das Polyurethangemisch zudem Polyvinylpyrrolidon (PVP), Polyvinylalkohol (PVA) oder auch andere hydrophile Polymere bzw. dazu geeignete Substanzen aufweisen kann. Der Anteil dieser Substanzen liegt dabei unter 10%, vorzugsweise unter 3%.

Insbesondere bei Kniespacern wird aufgrund der Konvexität der Kondylen des Femurs der zentrale Bereich des Gelenkspacers stark belastet. In Literatur und Patentschriften werden jedoch Kniespacer beschrieben, deren zentraler Bereich weicher und deren Randbereich härter ausgeführt sind. Allerdings neigen Kunststoffe zum Kriechen, wodurch der zentrale Bereich des Gelenkspacers bei dauernder Belastung "ausgebügelt" wird, also zentral wellig wird bzw. Falten werfen kann, die insgesamt zu Verwerfungen und zu Luxationen des Gelenkspacers führen können. Zudem können so die Randbereiche mit der Zeit immer weniger Last aufnehmen. Um einen Gelenkspacer mit den bevorzugten Dämpfungseigenschaften (Komplianz) zu erhalten, ist in einer weiteren Ausführungsform vorzugsweise zentral härteres und in den Randbereichen des Gelenkspacers weicheres Material angeordnet. Ein Meniskusspacer ist so ausgelegt, dass zuerst die weicheren Randbereiche durch den Femur und anschließend der härtere zentrale Bereich belastet werden, wodurch das progressive Druck-Stauchungsverhalten erreicht wird. Die femurale Seite des Meniskusspacers ist dabei konkaver ausgebildet als die Konvexität des Femurs, d.h. bei Kontakt werden zuerst die Randbereiche des Meniskusspacers durch den Femur berührt.

Vorzugsweise werden hierbei die zuvor aufgezeigten härteren bzw. weicheren Materialien mit den entsprechenden 50%-Zug- oder Druckspannungswerten eingesetzt. Diese Ausführung kann aber auch aus bekannten Polyurethanen aufgebaut sein. Als hartes Material vorzugsweise mit dem Shore-A-Härtebereich zwischen 78 und 85 kann beispielsweise ein Material mit den niedrigeren 50%-Zug- oder Druckspannungswerten des Shore-A-Härtebereichs bis 77 genutzt werden. Zudem kann als weiches Material für den Randbereich ein Material mit niedrigeren 50%-Zug- oder Druckspannungswerten als den angegebenen des Shore-A-Härtebereichs 20-77 eingesetzt werden. Vorzugsweise verlaufen die weichen Bereiche keilförmig von Rand des Meniskusspacers in die Mitte und sind beidseitig vom harten Material eingebettet, wobei zentral nur das harte Material vorliegt. Die Verformung der weichen Randbereiche und damit der Erhalt der Rückstellung dieser Bereiche werden begrenzt durch den zentralen harten Bereich, welcher die volle Last aufnimmt.

Weiterhin kann der Gelenkspacer einen schichtartigen Aufbau mit mindestens drei Schichten aufweisen, nämlich zwei Deckschichten und einer dazwischen angeordneten Kernschicht. Dabei bestehen die Kernschichten und die Deckschicht aus unterschiedlich harten Materialien, wobei vorzugsweise die Kernschicht aus einer vergleichsweise härteren und die Deckschichten aus einer vergleichsweise weicheren Schicht bestehen. Die relativ weichen Randbereiche sind vorzugsweise in ihren Dicken entsprechend so bemessen, dass zunächst diese Bereiche von einer Kondyle belastet werden, womit der Gelenkspacer zunächst eine ausnehmend hohe Komplianz aufweist. Mit zunehmender Stauchung der flexiblen Randbereiche verhält sich der Gelenkspacer aufgrund der vergleichsweise härteren Kernschicht steifer (progressives Druck-Stauchungsverhalten).

Vorzugsweise ist die Differenz der Shore-Härte zwischen Deck- und Kernschicht größer als 5 und vorzugsweise zwischen 10 und 25. Die härtere Kernschicht besteht vorzugsweise aus einem Polymer mit einer Shore-A-Härte von 78 bis 90, vorzugsweise von 80 bis 84. Demgegenüber besitzen die Deckschichten eine Shore-A-Härte zwischen 20 und 77 und bevorzugt zwischen 40 und 70. Die harten bzw. weichen Schichten weisen 50%-Zug- oder Druckspannungswerte auf wie in der Ausführungsform zuvor mit weichem Rand beschrieben.

Auf eine der beiden weichen Deckschichten, insbesondere auf die distale, die - im Falle eines Kniespacers - zur Tibia (Schienbein) weist, kann auch verzichtet werden, bevorzugt dann, wenn der Kniespacer auf der Tibia fixiert wird. Der Vorteil der zusätzlichen weichen Schicht besteht jedoch darin, dass sich diese besser an die individuelle Gelenk-Topografie des Patienten anpassen kann. Angemerkt sei ferner, dass das progressive Druck-/Stauchungsverhalten grundsätzlich auch erzielt werden kann, wenn die Kernschicht weich und die Deckschichten hart sind.

Ferner sind die in dieser Erfindung ausgeführten Materialausgestaltungen und Sandwichstrukturen auch für weitere Gelenkspacer nutzbar, die in anderen Gelenken innerhalb (Bandscheibe, Schulter, Fuß und Hand) oder außerhalb als Orthesen des menschlichen Körpers eingesetzt werden können. Sie können auch für Gelenke von Tieren eingesetzt werden, wie z.B. für Pferde. In härteren Ausführungen sind die Materialien auch als Sehnen- bzw. Bänderersatz wie z.B. als Kreuzbänder im Kniegelenk geeignet.

Konkrete Ausgestaltungen und weitere bevorzugte Ausführungsformen der vorliegenden Erfindung werden im Folgenden anhand des Diagramms und der Figur erläutert. Es zeigen:
Diagramm: Spannungs-Dehnungsverläufe,

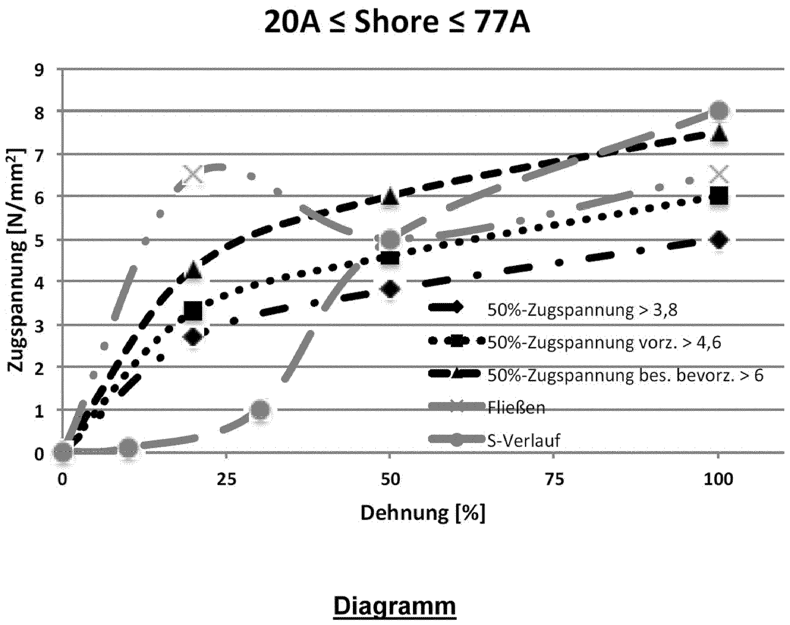

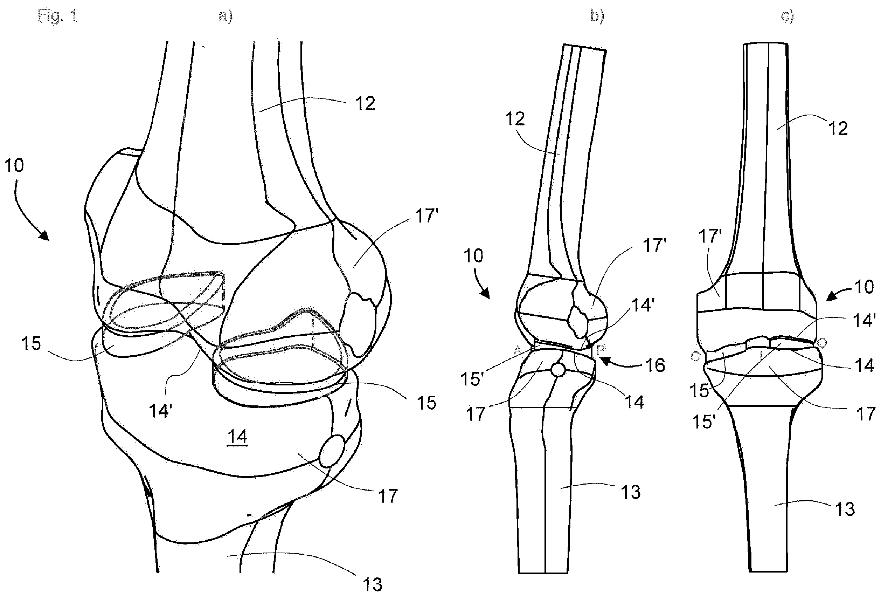

- Fig. 1a:: Tibia und Femur eines linken Knies mit medialem (links) und lateralem (rechts) Meniskusspacer und
- Fig. 1b, c:: eine Seitenansicht eines leicht gebeugten Knies von lateral (b) und in Frontalansicht (c),

Die im Folgenden benutzten Abkürzungen bedeuten folgendes:
- a: anterior
- p: posterior
- I: innere zur Kniemitte gelegene Seite eines Kniespacers
- O: äußere Seite eines Kniespacers

In Diagramm 1 ist der charakteristische Bereich der Zug-Spannungs-/Dehnungsverläufe der bevorzugten Materialien dargestellt. Es zeigt insbesondere die 50%-Zugspannungswerte für die Materialien im Shore-A-Härte Bereich 20-77, die größer 3,8 N/mm², vorzugsweise größer 4,6 N/mm² und besonders bevorzugt größer 6 N/mm² sein sollen. Das Diagramm legt zudem einen Spannungsverlauf mit einem Fließverhalten dar, welches vermieden werden sollte, es sei denn, die Fließgrenze würde sehr hohe Werte größer als 10N/mm² erreichen. Die S-Kurve ("S-Verlauf") verdeutlicht, dass ein geeignetes Material mit ausreichender 50%-Zugspannung sehr viel niedrigere Elastizitätsmodule und Zugspannungswerte bei Dehnungen unter 50% aufweisen kann wie in den anderen gezeigten Verläufen.

Die Fig. 1a-c zeigen unterschiedliche perspektivische Darstellungen eines (linken) Kniegelenkes 10 sowie Teilbereiche eines Oberschenkels 12 (Femur) und eines Unterschenkels 13 (Tibia). Zwischen den Gelenkflächen 14, 14' sind Meniskusspacer 15, 15' angeordnet, die den abgeriebenen und beschädigten (natürlichen) Meniskus ersetzen. Zur Materialschonung des Meniskusspacers 15, 15' müssen die zu übertragenden Kräfte auf eine möglichst große lastaufnehmende Fläche verteilt werden. Die Form, insbesondere die auf das Tibiaplateau 14 projizierte Randkontur eines Kniespacers 15, 15' spielt daher eine zentrale Rolle. Die höchsten Belastungen treten bei nahezu voll gestreckter Beinstellung auf, wie es in Fig. 1b angedeutet ist. In dieser leicht gebeugten Stellung ist aufgrund der im Verhältnis zum Tibiaplateau 14 nach posterior ansteigenden Femurkondyle und des sich dadurch vergrößernden Spaltes 16 die Beanspruchung in der anterioren Hälfte eines Kniespacer 15, 15' am höchsten. Zudem verschmälert sich die Auflagefläche des Tibiaplateaus 14 von anterior nach posterior durch eine Einmuldung (der Area intercondylaris posterior). Die posteriore Hälfte der Auflagefläche dient besonders zum Abrollen der Kondylen 17, 17' in stärker gebeugten Stellungen, in denen geringere Belastungen auftreten.

## Patentansprüche

1. Biokompatibles Polymer für Gelenkspacer, Implantate und/oder als Sehnen- oder Bänderersatz bestehend aus einem vernetzten Polyurethan, wobei das Polyurethan Polyurethanharnstoff oder Polyharnstoff ist,
**dadurch gekennzeichnet, dass**
zur Vernetzung des Polyurethans ein Vernetzungsreagenz eingesetzt wird, das zumindest anteilig Wasser aufweist.

2. Biokompatibles Polymer nach Anspruch 1, **dadurch gekennzeichnet, dass** das Vernetzungsreagenz einen oder mehrere Kettenverlängerer aufweist.

3. Biokompatibles Polymer nach einem der Ansprüche 1 oder 2, **dadurch gekennzeichnet, dass** das Vernetzungsreagenz ein oder mehrere Vernetzungsreagenzien aufweist.

4. Biokompatibles Polymer nach einem der Ansprüche 1 bis 3, **dadurch gekennzeichnet, dass** das Polymer zumindest teilweise aus einem Material, mit Zugspannungswerten bei Dehnungen zwischen 20% und 60%, vorzugsweise bei 50%, größer 3,8 N/mm², vorzugsweise größer 4,6 N/mm² und besonders bevorzugt größer 6 N/mm², und/oder Druckspannungswerten bei Stauchungen zwischen 20% und 60%, vorzugsweise bei 50%, größer 7,8 N/mm², vorzugsweise größer 9 N/mm² und besonders bevorzugt größer 10,5 N/mm² besteht.

5. Biokompatibles Polymer nach einem der Ansprüche 1 bis 4, **dadurch gekennzeichnet, dass** das Polymer als Weichsegmente hydroxyl- und/oder aminterminiertes Polyisobutylen, und/oder, hydroxyl- und/oder aminterminiertes und bevorzugt hydroxylterminiertes hydrogeniertes Polybutadien ausschließlich enthält.

6. Biokompatibles Polymer nach einem der Ansprüche 1 bis 5, **dadurch gekennzeichnet,**
a) **dass** das Polymer als Weichsegmente Polyisobutylen und/oder Polybutadien und ein Polycarbonatdiol und bevorzugt ein Polycarbonatdiol-Copolymertyp mit einem Anteil des Polycarbonatdiol am Gesamtweichsegmentanteil von bis zu 80%, vorzugsweise 5 bis 40%, enthält, und/oder
b) **dass** das Weichsegment oder das Gemisch der Weichsegmente des Polymers die Weichsegmente Polydimethylsiloxan oder Polytetramethylenoxid oder Polydimethylsiloxan-Polycaprolacton-Blockcopolymere oder ein Gemisch einzelner oder mehrerer dieser Weichsegmente enthalten, und zwar mit einem Anteil eines oder mehrerer dieser Weichsegmente am Gesamtweichsegmentanteil von bis zu 50%, vorzugsweise von 3 bis 35%.

7. Biokompatibles Polymer nach einem der Ansprüche 1 bis 6, **dadurch gekennzeichnet, dass** das Polyurethan zumindest teilweise aus Naphthalin-1,5-Diisocyanat und/oder Para-Phenylendiisocyanat und/oder trans-1,4-Cyclohexandiisocyanat und/oder Hexamethylendiisocyanat besteht.

8. Biokompatibles Polymer nach einem der Ansprüche 1 bis 7, **dadurch gekennzeichnet, dass** das Polymer als Gelenkspacer ausgebildet ist, wobei
a) die Randzone des Gelenkspacers zumindest teilweise aus einem Material mit einer Shore-A-Härte zwischen 20 und 77 besteht und das Material des zentralen (härteren) Bereichs eine Shore-A-Härte größer 77 mit Zugspannungswerten bei Dehnungen zwischen 20% und 60%, vorzugsweise bei 50%, größer 3,8 N/mm², vorzugsweise größer 4,6 N/mm² und besonders bevorzugt größer 6 N/mm², und/oder Druckspannungswerten bei Stauchungen zwischen 20% und 60 %, vorzugsweise bei 50%, größer 7,8 N/mm², vorzugsweise größer 9 N/mm² und besonders bevorzugt größer 10,5 N/mm² aufweist, oder
b) der Gelenkspacer einen zumindest teilweise schichtartigen Aufbau mit mindestens drei Schichten aufweist, nämlich mindestens zwei Deckschichten und einer dazwischen angeordneten Kernschicht, die aus unterschiedlich harten Materialien bestehen, wobei die Differenz der Shore-A-Härten zwischen Deck- und Kernschicht größer als 5 und vorzugsweise zwischen 10 und 20 liegt und zumindest eine der Schichten aus einem Material mit Zugspannungswerten bei Dehnungen zwischen 20% und 60 %, vorzugsweise bei 50%, größer 3,8 N/mm², vorzugsweise größer 4,6 N/mm² und besonders bevorzugt größer 6 N/mm² und/oder Druckspannungswerten bei Stauchungen zwischen 20% und 60 %, vorzugsweise bei 50%, größer 7,8 N/mm², vorzugsweise größer 9 N/mm² und besonders bevorzugt größer 10,5 N/mm² besteht.

## Claims

1. Biocompatible polymer for joint spacers, implants and/or as a tendon or ligament replacement, consisting of a crosslinked polyurethane, the polyurethane being polyurethaneurea or polyurea,
**characterized in that**
a crosslinking reagent is used to crosslink the polyurethane, which contains at least a proportion of water.

2. Biocompatible polymer according to claim 1, **characterized in that** the crosslinking reagent comprises one or more chain extenders.

3. Biocompatible polymer according to one of claims 1 or 2, **characterized in that** the crosslinking reagent comprises one or more crosslinking reagents.

4. Biocompatible polymer according to one of claims 1 to 3, **characterized in that** the polymer consists at least partly of a material with tensile stress values at elongations between 20% and 60%, preferably at 50%, greater than 3.8 N/mm², preferably greater than 4.6 N/mm² and particularly preferably greater than 6 N/mm², and/or compressive stress values at compressions between 20% and 60%, preferably at 50%, greater than 7.8 N/mm², preferably greater than 9 N/mm² and particularly preferably greater than 10.5 N/mm².

5. Biocompatible polymer according to one of claims 1 to 4, **characterized in that** the polymer exclusively contains hydroxyl- and/or amine-terminated polyisobutylene and/or hydroxyl- and/or amine-terminated and preferably hydroxyl-terminated hydrogenated polybutadiene as soft segments.

6. Biocompatible polymer according to one of claims 1 to 5, **characterized in that**
a) that the polymer contains as soft segments polyisobutylene and/or polybutadiene and a polycarbonate diol and preferably a polycarbonate diol copolymer type with a proportion of the polycarbonate diol in the total soft segment proportion of up to 80%, preferably 5 to 40%, and/or
b) that the soft segment or mixture of soft segments of the polymer contains the soft segments polydimethylsiloxane or polytetramethylene oxide or polydimethylsiloxane-polycaprolactone block copolymers or a mixture of one or more of these soft segments, with a proportion of one or more of these soft segments in the total soft segment proportion of up to 50%, preferably from 3 to 35%.

7. Biocompatible polymer according to one of claims 1 to 6, **characterized in that** the polyurethane consists at least partly of naphthalene-1,5-diisocyanate and/or para-phenylene diisocyanate and/or trans-1,4-cyclohexane diisocyanate and/or hexamethylene diisocyanate.

8. Biocompatible polymer according to one of claims 1 to 7, **characterized in that** the polymer is formed as a joint spacer, wherein
a) the edge zone of the joint spacer consists at least partly of a material with a Shore A hardness between 20 and 77 and the material of the central (harder) region has a Shore A hardness greater than 77 with tensile stress values at elongations between 20% and 60%, preferably at 50%, greater than 3.8 N/mm², preferably greater than 4.6 N/mm² and particularly preferably greater than 6 N/mm², and/or compressive stress values at compression between 20% and 60%, preferably at 50%, greater than 7.8 N/mm², preferably greater than 9 N/mm² and particularly preferably greater than 10.5 N/mm², or
b) the joint spacer has an at least partially layered structure with at least three layers, namely at least two cover layers and a core layer arranged between them, which consist of materials of different hardnesses, the difference in Shore A hardnesses between the cover and core layers being greater than 5 and preferably between 10 and 20 and at least one of the layers consisting of a material with tensile stress values at elongations of between 20% and 60%, preferably at 50%, greater than 3.8 N/mm², preferably at 50%, preferably at 3.8 N/mm² and particularly preferably greater than 10.5 N/mm², or preferably at 50%, greater than 3.8 N/mm², preferably greater than 4.6 N/mm² and particularly preferably greater than 6 N/mm² and/or compressive stress values at compression between 20% and 60%, preferably at 50%, greater than 7.8 N/mm², preferably greater than 9 N/mm² and particularly preferably greater than 10.5 N/mm².

## Revendications

1. Polymère biocompatible pour des spacer articulaire, des implants et/ou comme remplacement de tendons ou de ligaments, constitué d'un polyuréthane réticulé, le polyuréthane étant de la polyuréthane-urée ou de la polyurée,
**caractérisé en ce que** pour la réticulation du polyuréthane, on utilise un réactif de réticulation qui contient au moins en partie de l'eau.

2. Polymère biocompatible selon la revendication 1, **caractérisé en ce que** le réactif de réticulation comprend un ou plusieurs agents d'allongement de chaîne.

3. Polymère biocompatible selon l'une des revendications 1 ou 2, **caractérisé en ce que** le réactif de réticulation comprend un ou plusieurs agents de réticulation.

4. Polymère biocompatible selon l'une des revendications 1 à 3, **caractérisé en ce que** le polymère est constitué au moins en partie d'un matériau présentant des valeurs de contraintes de traction en allongement comprises entre 20% et 60%, de préférence à 50%, supérieures à 3,8 N/mm², de préférence supérieures à 4,6 N/mm² et de manière particulièrement préférée supérieures à 6 N/mm², et/ou des valeurs de contraintes de compression en compression comprises entre 20% et 60%, de préférence à 50%, supérieures à 7,8 N/mm², de préférence supérieures à 9 N/mm² et de manière particulièrement préférée supérieures à 10,5 N/mm².

5. Polymère biocompatible selon l'une quelconque des revendications 1 à 4, **caractérisé en ce que** le polymère contient exclusivement, en tant que segments mous, du polyisobutylène à terminaison hydroxyle et/ou amine, et/ou du polybutadiène hydrogéné à terminaison hydroxyle et/ou amine et de préférence à terminaison hydroxyle.

6. Polymère biocompatible selon l'une des revendications 1 à 5, **caractérisé en ce que** ,
a) que le polymère contient comme segments mous du polyisobutylène et/ou du polybutadiène et un polycarbonatediol et de préférence un type de copolymère de polycarbonatediol avec une proportion de polycarbonatediol dans la proportion totale de segments mous allant jusqu'à 80%, de préférence de 5 à 40%, et/ou
b) **en ce que** le segment mou ou le mélange de segments mous du polymère contient les segments mous polydiméthylsiloxane ou polytétraméthylène oxyde ou copolymères séquencés polydiméthylsiloxane-polycaprolactone ou un mélange d'un ou plusieurs de ces segments mous, et ce avec une proportion d'un ou plusieurs de ces segments mous dans la proportion totale de segments mous allant jusqu'à 50%, de préférence de 3 à 35%.

7. Polymère biocompatible selon l'une quelconque des revendications 1 à 6, **caractérisé en ce que** le polyuréthane est constitué au moins en partie de naphtalène-1,5-diisocyanate et/ou de para-phénylènediisocyanate et/ou de trans-1,4-cyclohexanediisocyanate et/ou d'hexaméthylènediisocyanate.

8. Polymère biocompatible selon l'une des revendications 1 à 7, **caractérisé en ce que** le polymère est conçu comme un spacer articulaire, dans lequel
a) la zone marginale du spacer articulaire est constituée au moins partiellement d'un matériau ayant une dureté Shore A comprise entre 20 et 77 et le matériau de la zone centrale (plus dure) a une dureté Shore A supérieure à 77 avec des valeurs de contrainte de traction pour des allongements comprises entre 20% et 60%, de préférence à 50%, supérieure à 3,8 N/mm², de préférence supérieure à 4,6 N/mm² et de manière particulièrement préférée supérieure à 6 N/mm², et/ou des valeurs de contrainte de compression pour des compressions entre 20% et 60%, de préférence à 50%, supérieures à 7,8 N/mm², de préférence supérieures à 9 N/mm² et de manière particulièrement préférée supérieures à 10,5 N/mm², ou bien
b) le spacer articulaire présente une structure au moins partiellement stratifiée avec au moins trois couches, à savoir au moins deux couches de recouvrement et une couche centrale disposée entre elles, qui sont constituées de matériaux de dureté différente, la différence des duretés Shore A entre la couche de recouvrement et la couche centrale étant supérieure à 5 et de préférence comprise entre 10 et 20, et au moins l'une des couches étant constituée d'un matériau présentant des valeurs de contrainte de traction pour des allongements compris entre 20 % et 60 %, de préférence à 50%, supérieur à 3,8 N/mm², de préférence supérieur à 4,6 N/mm² et de manière particulièrement préférée supérieur à 6 N/mm² et/ou des valeurs de contrainte de compression pour des écrasements entre 20% et 60%, de préférence à 50%, supérieur à 7,8 N/mm², de préférence supérieur à 9 N/mm² et de manière particulièrement préférée supérieur à 10,5 N/mm².
